(19) Europäisches Patentamt
European Patent Office
Office européen des brevets

(11) **EP 4 268 710 A1**

(12) **EUROPÄISCHE PATENTANMELDUNG**

(43) Veröffentlichungstag:
**01.11.2023  Patentblatt 2023/44**

(21) Anmeldenummer: **23152373.9**

(22) Anmeldetag: **19.01.2023**

(51) Internationale Patentklassifikation (IPC):
*A61B 5/00* (2006.01)      *A61B 5/374* (2021.01)

(52) Gemeinsame Patentklassifikation (CPC):
**A61B 5/374; A61B 5/4818; A61B 5/6803**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **27.04.2022  EP 22170323**

(71) Anmelder: **Universitätsmedizin der Johannes Gutenberg-Universität Mainz 55131 Mainz (DE)**

(72) Erfinder:
• **GOUVERIS, Haralampos 55131 Mainz (DE)**
• **MUTHURAMAN, Muthuraman 55234 Freimersheim (DE)**

(74) Vertreter: **Patentanwälte Dr. Keller, Schwertfeger Partnerschaft mbB Westring 17 76829 Landau (DE)**

(54) **VERFAHREN ZUR BESTIMMUNG EINES MASSES FÜR DEN GRAD EINER OBSTRUKTIVEN SCHLAFAPNOE UND/ODER DEREN FOLGEERSCHEINUNG, WIE ETWA DER TAGESSCHLÄFRIGKEIT**

(57)    Die vorliegende Erfindung betrifft ein Verfahren zur Bestimmung eines Maßes für den Grad einer obstruktiven Schlafapnoe und/oder deren Folgeerscheinung mittels folgender Schritte:
- Festlegen eines Maßes für den Grad einer obstruktiven Schlafapnoe und/oder deren Folgeerscheinung,
- Bereitstellung zweier EEG-Messsignale einer Elektroenzephalographie an den Elektroenzephalographiestellen eines 10-20 internationalen EEG Systems sind,
- Aufteilen der EEG- Messsignale in Frequenzbänder,
- Bestimmen wenigstens eines Kreuzfrequenzmodulationsindex mittels Daten aus wenigstens zwei verschiedenen Frequenzbändern,
- Bestimmen des Maßes für den Grad einer obstruktiven Schlafapnoe und/oder dessen Folgeerscheinung mittels des wenigstens einen Kreuzfrequenzmodulationsindex.
    Ferner betrifft die Erfindung eine Vorrichtung zur Durchführung des Verfahrens.

Fig. 1

**Beschreibung**

Die vorliegende Erfindung betrifft ein Verfahren zur Bestimmung eines Maßes für den Grad einer obstruktiven Schlafapnoe und/oder deren Folgeerscheinung, wie etwa der Tagesschläfrigkeit.

Eine Schlafapnoe ist eine nächtliche Atemstörung, bei der es im Schlaf immer wieder zu längeren Atemstillständen oder Teilatemstillständen kommt. Unter längeren Atemstillständen oder längeren Teilatemstillständen versteht man per Definition Atemstillstände oder Teilatemstillstände, die länger als 10 Sekunden dauern. Kurze, weniger als 10 Sekunden andauernde Atemaussetzer werden in der Regel als nicht gesundheitsschädlich angesehen. Sollte es während des Schlafs jedoch häufiger als fünf Mal pro Stunde Schlaf zu längeren Atemaussetzern oder Teilatemaussetzern kommen, kann dies schwere gesundheitsschädliche Folgen haben.

Obstruktive Schlafapnoe (OSA) ist bei Erwachsenen weit verbreitet. Die Störung kann eine neuronale Schädigung verursachen oder OSA selbst kann eine neurologische Störung sein. Die Stimulation des Nervus hypoglossalis, die bei vielen OSA-Patienten als wirksame Behandlung dient, unterstützt das Argument, dass OSA eine neuronale / neuromuskuläre Störung ist. Dementsprechend wurde bei OSA ein Hinweis auf eine Trennung zwischen motorisch assoziierten kortikalen neuronalen Gruppen und peripheren oberen zervikalen motorischen Einheiten gefunden. Darüber hinaus gibt es Hinweise aus funktionellen MRT-Studien für eine Trennung zwischen sensomotorischen und anderen kortikalen Regionen bei OSA.

Schläfrigkeit, eine häufiger von Patienten berichtete Auswirkung bei OSA, weist signifikante Verbindungen mit funktioneller Konnektivität innerhalb des sensomotorischen Netzwerks auf. Eine funktionelle Neuroimaging-Studie an Erwachsenen über 55 Jahren zeigte eine Hypoperfusion in sensomotorischen Bereichen von Patienten mit schwerer OSA. Darüber hinaus haben Neuroimaging-Studien zur interhemisphärischen Interaktion einen starken Zusammenhang zwischen Schläfrigkeit und der Aktivität des bilateralen präzentralen Gyrus, einem wichtigen Teil des sensomotorischen Netzwerks, nach Schlafentzug festgestellt.

Üblicherweise wird eine Schlafapnoe bzw. der Schweregrad einer Schlafapnoe in einem Schlaflabor mit Hilfe einer kardiorespiratorischen Polysomnographie festgestellt. Der Schweregrad wird hierbei aktuell in drei Schweregradgruppen eingeteilt.

Über eine bzw. zwei aufeinander folgende Nächte werden Messsensoren an den Patienten angebracht, der an einer obstruktiven Schlafapnoe erkrankt ist. Es erfolgt über ca. 8 Stunden eine Aufzeichnung von 18 verschieden physiologischen Signalen: Elektroenzephalogram (EEG, 4 Signale), Elektrookulogram (EOG, 2 Signale), Elektromyogram (EMG, 3 Signale) am Kinn und an beiden Unterschenkeln, Elektrokardiogram (EKG, 1 Signale), Messung des Pulsschlags und der Sauerstoffsättigung im Blut (Pulsoximetrie, 2 Signale), Atemflussmessung über Nase und Mund (2 Sign.), Atemanstrengung am Bauch und Brustkorb (2 Signale), Schnarchmikrofon am Hals (1 Signal) und Körperlage (1 Signal).

Im Rahmen der kardiorespiratorischen Polysomnographie (KRPSG) erfolgt eine Berechnung des Schweregrades der OSA mit Hilfe des RDI (Respiratory Disturbance Index), d.h. der Anzahl der Apnoen, Hypopnoen und sog. RERAs (respiratory - effort related arousals) pro Stunde Schlaf. Menschen mit RDI > 15/ Std. Schlaf haben eine klinisch signifikante OSA und sollen nach aktuellen Leitlinien zeitnah behandelt werden. Menschen mit RDI = 5-15/Std. haben eine geringgradige OSA und sollen nicht unbedingt zeitnah behandelt werden und Menschen mit RDI < 5/Std sind gesund (leiden unter keiner OSA).

Die Epworth Schläfrigkeitsskala (Epworth Sleepiness Scale, ESS) dient zur subjektiven Einschätzung der Tagesschläfrigkeit bei Patienten mit OSA (Johns 1991). Es handelt sich um einen Fragebogen mit 8 Fragen zur subjektiven (vom Patienten selbst empfundenen) Einschätzung der Neigung zur Tagesschläfrigkeit in monotonen Alltagssituationen (keine Neigung = 0 Punkte; maximale Neigung = 3 Punkte; d.h. die kumulative Punktzahl kann zwischen 0 und 24 Punkte variieren). Bei einer ESS-Gesamtpunktzahl > 10 ist die Wahrscheinlichkeit zur signifikanten, klinisch relevanten, Tagesschläfrigkeit sehr erhöht.

Die Auswertung der Messung erfolgt danach automatisch oder manuell über die in den kommerziell verfügbaren KRPSG-Software-Systemen eingebetteten Algorithmen, die acht der oben genannten 18 Signale der KRPSG für die Berechnung des RDI verwenden. Zur Qualitätssicherung ist dabei unbedingt notwendig, dass eine visuelle Auswertung seitens eines Experten (Pfleger, medizinisch-technischer Assistent, Arzt), jeweils mit einem variierenden Ausbildungsniveau, erfolgen muss.

Für die Berechnung der ESS ist eine subjektive Einschätzung der Tagesschläfrigkeit mit Hilfe des ESS-Fragebogens notwendig.

Dieses Verfahren ist vergleichsweise aufwändig.

Es ist Aufgabe der vorliegenden Erfindung, ein möglichst automatisiertes Verfahren zur Erfassung und speziell zur Auswertung von Messdaten bereitzustellen, welches es ermöglicht, auf Basis der Daten auf einfache Weise eine Aussage über ein Maß für den Schweregrad der OSA sowie das Ausmaß der verbundenen subjektiv eingeschätzten Tagesschläfrigkeit bei den OSA-Patienten auf einfache Art und Weise treffen zu können, insbesondere eine entsprechende Aussage auf einfache Art und Weise ohne Mitwirkung von Experten treffen zu können.

Die Aufgabe wird durch ein Verfahren zur Bestimmung eines Maßes für den Grad einer obstruktiven Schlafapnoe und/oder deren Folgeerscheinung mittels einer Auswertung von Messdaten gemäß Anspruch 1 gelöst, bei dem zunächst ein Maß für den Grad einer obstruktiven Schlafapnoe und/oder deren Folgeerscheinung festgelegt wird, danach werden zwei EEG-Messsignale einer Elektroenzephalographie an den Elektroenzephalographiestellen eines 10-20 internationalen EEG-Systems bereitgestellt. Die EEG-Messsignale werden in Frequenzbänder aufgeteilt, insbesondere entsprechend gefiltert. Aus den Daten wenigstens zweier verschiedener Frequenzbänder wird wenigstens ein Kreuzfrequenzmodulationsindex bestimmt. Anschließend wird das Maß für den Grad einer obstruktiven Schlafapnoe und/oder deren Folgeerscheinung mittels des wenigstens einen Kreuzfrequenzmodulationsindex bestimmt.

Bei diesem Verfahren handelt es sich somit um die Bereitstellung und Auswertung von nur zwei EEG-Signalen. Das beanspruchte Verfahren generiert Daten, die für eine Korrelation mit einem Maß für den Grad einer obstruktiven Schlafapnoe und/oder deren Folgeerscheinung geeignet sind, so dass eine Aussage über den Grad einer obstruktiven Schlafapnoe und/oder deren Folgeerscheinung getroffen werden kann. Die Bereitstellung sowie die Auswertung der Daten können vollautomatisiert durchgeführt werden.

Ein Maß für den Schweregrad der obstruktiven Schlafapnoe stellt beispielsweise der Respiratory Disturbance Index dar. Eine Folgeerscheinung einer obstruktiven Schlafapnoe ist die Tagesschläfrigkeit. Der Grad der Tagesschläfrigkeit wird beispielsweise mittels der Epworth Schläfrigkeitsskala (ESS) angegeben.

Bei dem 10-20 internationalen EEG-System handelt es sich um eine international anerkannte Methode zur Beschreibung der Position von Kopfhautelektroden im Rahmen einer EEG-Messung, insbesondere einer Polysomnographie-Schlafstudie.

Besonders vorteilhaft ist es, wenn die EEG- Messignale während des Schlafens zuhause ermittelt werden. Es besteht jedoch auch die Möglichkeit, die Daten in einem Schlaflabor oder in einer Arztpraxis zu erfassen.

Das Verfahren ist besonders einfach umzusetzen, wenn gemäß einer bevorzugten Ausführungsform das Maß für den Grad einer obstruktiven Schlafapnoe und/oder dessen Folgeerscheinung, insbesondere der Respiratory Disturbance Index und/oder die Tagesschläfrigkeit nur auf Basis der beiden EEG- Messsignale bestimmt wird.

Als besonders vorteilhaft hat es sich erwiesen, dass die Elektroenzephalographiestellen, an denen die EEG-Messsignale erfasst werden, die Stellen C3 und C4 sind. Mit Hilfe der an diesen Stellen gewonnenen Messdaten lässt sich der Respiratory Disturbance Index bzw. ein Maß für die Tagesschläfrigkeit mit vergleichsweise hoher Genauigkeit bestimmen.

Es ist von Vorteil, dass die Aufteilung der Messignale in Frequenzbänder für jede Messstelle getrennt erfolgt, um Messstörungen an einer Messstelle auszuschließen.

Der Erfindung liegen folgende Erkenntnisse zugrunde:
Die Kreuzfrequenzkopplung (CFC) ist ein grundlegendes Merkmal der oszillatorischen Aktivität des Gehirns und korreliert stark mit der Gehirnfunktion. CFC umfasst verschiedene Muster: Phasensynchronisation; Amplituden-Co-Modulation und Phasen-Amplituden-Kopplung (PAC). In der vorliegenden Erfindung wurden PAC untersucht, da es neuronale Kodierung und Informationsübertragung innerhalb lokaler mikroskaliger und makroskaliger neuronaler Ensembles des Gehirns darstellt. Niederfrequente oszillierende Aktivität stellt die Regulation des Informationsflusses zwischen Gehirnarealen dar, indem sie die Erregbarkeit lokaler Gehirnensembles moduliert. Die Phase dieser niederfrequenten oszillatorischen Aktivität beeinflusst sowohl den Rhythmus der Hochfrequenzaktivität als auch die Feuerrate einzelner Neuronen. Daher scheint die Phasen-Amplituden-Kreuzfrequenzkopplung (PACFC, phase-amplitude cross-frequency coupling) eine effektive Interaktion zwischen Neuronen ähnlicher Phasenpräferenzen und die Synchronisation von Hochfrequenzbändern während spezifischer langsamerer Rhythmusphasen zu fördern.

Das Verständnis neurophysiologischer Konnektivitätsmuster und der oszillatorischen Dynamik an den sensomotorischen kortikalen Bereichen kann dazu beitragen, pathophysiologische Prozesse des Zentralnervensystems (ZNS) in OSA weiter aufzuklären. Angesichts der Tatsache, dass OSA klinisch mit kognitiven, Wachsamkeits- und Wachheitsstörungen im Wachzustand assoziiert ist (insbesondere übermäßige Tagesschläfrigkeit), kann ein besseres Verständnis und eine bessere Modellierung einer solchen Konnektivität die Basis für die Vorhersage relevanter klinischer Phänomene schaffen.

Im Rahmen dieser Erfindung wird die funktionelle Netzwerkkonnektivität des sensomotorischen Bereichs bei therapienaiven OSA-Patienten charakterisiert. Dazu wurde zunächst getestet, ob sich die schlafstadienspezifische Theta-gamma-PACFC-Modulation zwischen Patienten mit und ohne signifikanter OSA unterscheidet. Diese Bewertung basierte auf den Ergebnissen früherer Studien, die eine signifikante Rolle der Theta-Oszillation während verschiedener Schlafstadien gezeigt haben. Darüber hinaus wurde untersucht, ob diese Modulationen spezifisch für ein bestimmtes Schlafstadium waren. Um zu beurteilen, ob eine mögliche funktionelle Trennung im sensomotorischen Bereich frequenzspezifisch war, wurde als Kontrollexperiment die Delta-alpha-PACFC-Modulation zwischen den Patienten mit und ohne signifikante OSA verglichen. Schließlich wurde untersucht, ob eine solche Trennung mit klinischen Parametern wie den von Patienten berichteten Schläfrigkeitsergebnissen korreliert war (Epworth Sleepiness Scale; ESS).

Frühere Studien haben die Kreuzfrequenzkopplung für die Schlafstadienklassifizierung vor allem bei gesunden Erwachsenen, aber auch bei OSA-Patienten verwendet. Keine dieser Studien bewertet jedoch die Bedeutung dieser Klassifizierung in Bezug auf die klinische Übertragbarkeit für OSA-Patienten. Bei dieser Erfindung werden die frequenzspezifischen CFC-basierten Modulationsindizes nicht nur für die Schlafstadienklassifizierung verwendet, sondern zeigen weiterhin, dass die gleichen Modulationsindizes auch klinische Scores wie ESS vorhersagen könnten. Daher sollten diese Ergebnisse helfen zu enträtseln, ob entweder eine frequenzspezifische, schlafstadienspezifische oder globale funktionelle Trennung im sensomotorischen Bereich von OSA-Patienten besteht. Darüber hinaus kann dies einen objektiven neurophysiologischen Surrogatmarker liefern, um die vom Patienten berichtete subjektive Schläfrigkeit sowie die Schwere der Atemwegserkrankung bei OSA-Patienten zu quantifizieren.

Bei einer bevorzugten Ausführungsform des Verfahrens wird daher der wenigstens eine Kreuzfrequenzmodulationsindex mittels einer Phase-Amplituden Kreuzfrequenzkopplung ermittelt. Hierfür werden vorzugsweise die Messsignale in wenigstens zwei der folgenden Frequenzbänder aufgeteilt: niedriges Frequenzband von 0,1 bis 1 Hz, Delta-Band (1 bis 3 Hz), Theta-Band (4 bis 7 Hz), Alpha-Band (8-13 Hz), Beta-Band (14 bis 30 Hz) und Gamma-Band (31-100 Hz).

Insbesondere werden die beiden Messignale in die beiden Frequenzbänder Alpha-Band (8-13 Hz) und Delta-Band (1 bis 3 Hz) aufgeteilt, wobei vorzugsweise mittels einer Phase-Amplituden-Kreuzfrequenzkopplung aus dem Alpha-Band die Amplituden- Hüllkurve und aus dem Delta-Band die Phase der Messignale bestimmt werden.

Zusätzlich oder alternativ werden die Messsignale in die beiden Frequenzbänder Theta-Band (4 bis 7 Hz) und Gamma-Band (31- 100 Hz) aufgeteilt, wobei vorzugsweise mittels einer Phase-Amplituden Kreuzfrequenzkopplung aus dem Gamma-Band die Amplituden- Hüllkurve und aus dem Theta-Band die Phase der Messignale bestimmt werden.

Die für das Verfahren benötigten EEG Messignale sind vorzugsweise in einer Datenbank oder einem Speichermodul gespeichert, so dass sie zeitlich und räumlich unabhängig von der Erfassung der EEG-Messsignale zur Verfügung stehen. Dies ermöglicht beispielsweise die Erstellung einer Korrelationsdatenbank, bei der ein Maß für den Grad einer obstruktiven Schlafapnoe und/oder deren Folgeerscheinung mit dem Kreuzfrequenzmodulationsindex korreliert ist.

Bei einer bevorzugten Ausführungsform wird eine Korrelationsdatenbank mit Korrelationsdaten zwischen dem wenigstens einen Kreuzfrequenzmodulationsindex und dem Maß für den Grad einer obstruktiven Schlafapnoe und/oder dessen Folgeerscheinung bereitgestellt, wobei dann das Maß für den Grad einer obstruktiven Schlafapnoe und/oder dessen Folgeerscheinung mit Hilfe der Daten der Korrelationsdatenbank aus dem wenigstens einen Kreuzfrequenzmodulationsindex bestimmt wird.

Die Bereitstellung einer Korrelationsdatenbank ermöglicht eine besonders einfache und genaue Zuordnung des Kreuzfrequenzmodulationsindex einem Maß für den Grad einer obstruktiven Schlafapnoe und/oder dessen Folgeerscheinung.

Vorzugsweise werden das Maß für den Grad einer obstruktiven Schlafapnoe und/oder dessen Folgeerscheinung, insbesondere der Respiratory Disturbance Index und/oder die Tagesschläfrigkeit mittels einer Support Vector Machine auf Basis des wenigstens einen Kreuzfrequenzmodulationsindex bestimmt.

Zusammengefasst zeigt die Phase-Amplituden-Kreuzfrequenz-Kopplung die Modulation der Hochfrequenzleistung im C3-/C4- EEG-Signal durch die Niederfrequenzphase im gleichen (C3-/C4-) EEG-Signal an. Dieser crossfrequency coupling (CFC) - Modulationsindex (MI) wird dann verwendet, um die Phasen-Amplituden-Zuordnung zwischen phasenmodulierendem Frequenzband (z.B. Delta) und amplitudenmodulierten Frequenzbändern (z.B. Gamma) zu identifizieren. Für die Messung des frequenzübergreifenden Kopplungsmaßes werden nur die Signale verwendet, die vom Gehirn aufgezeichnet wurden, nämlich die Signale aus den C3- und C4- Elektroden. Nach der Schätzung des MI wird der Support-Vector-Machine-Learning-Algorithmus zur Vorhersage des RDI sowie der ESS (Epworth-Schläfrigkeitsskala) verwendet.

Bei einer bevorzugten Weiterbildung des beschriebenen Verfahrens werden die Messsignale während des Schlafs erfasst und der Schlaf in Schlafstadien eingeteilt, wobei der wenigstens eine Kreuzfrequenzmodulationsindex in Abhängigkeit von Schlafstadien bestimmt wird, so dass eine Zuordnung des Kreuzfrequenzmodulationsindex und des Maßes für den Grad einer obstruktiven Schlafapnoe und/oder deren Folgeerscheinung in Abhängigkeit von Schlafstadien angegeben werden kann. Hierdurch lassen sich sehr präzise Aussagen über den Schwergrad eine Schlafapnoe und der Tagesschläfrigkeit treffen, denn es wurde festgestellt, dass in Abhängigkeit vom Schweregrad der Schlafapnoe und/oder der Tagesschläfrigkeit die einzelnen Kreuzfrequenzmodulationsindizes sich in den einzelnen Schlafstadien unterscheiden.

Es ist von besonderem Vorteil, dass alle Verfahrensschritte automatisch durchgeführt werden, wobei hier vorteilhafterweise bei einer Erfassung der Messignale im Schlaf eine automatische Klassifikation der Schlafstadien durchgeführt wird.

Gegenstand der vorliegenden Erfindung ist auch eine Vorrichtung zur Durchführung eines Verfahrens zur Bestimmung eines Maßes für den Grad einer obstruktiven Schlafapnoe und/oder deren Folgeerscheinung, insbesondere eine Vorrichtung zur Durchführung des beschriebenen Verfahrens, wobei die Vorrichtung eine Kopfbedeckung mit zwei Sensoren zur Erfassung der EEG-Signale, insbesondere an den Stellen C3 und C4 umfasst.

Da für die Durchführung des erfindungsgemäßen Verfahrens nur zwei Messstellen notwendig sind, ist es ausreichend, dass die Kopfbedeckung nur zwei Sensoren zur Bestimmung der EEG-Signale, insbesondere an den Stellen C3 und C4 umfasst.

Weiterhin können ein Datenspeicher und ein Datenbearbeitungsmodul vorgesehen sein. Der Datenspeicher ist vorgesehen, um die EEG-Signale zu speichern und für eine weitere Auswertung zur Verfügung zu stellen. Das Datenbearbeitungsmodul ist vorgesehen, um aus den Daten einen Kreuzfrequenzmodulationsindex gemäß der vorliegenden Erfindung zu bestimmen.

Das Datenbearbeitungsmodul ist darüber hinaus ausgelegt, aus dem

Kreuzfrequenzmodulationsindex ein Maß für den Grad einer obstruktiven Schlafapnoe und/oder dessen Folgeerscheinung zu bestimmen.

Bei einer bevorzugten Ausführungsform ist die Kopfbedeckung eine Mütze, eine Kappe, wobei die Kappe zusätzlich ein Kinnteil aufweisen kann, oder ein Stirnband.

Die Erfindung hat den Vorteil, dass die Bestimmung des RDI sowie des ESS unter Nutzung allein von zwei EEG-Elektroden (statt der standardmäßig verwendeten 8 der o.g. 18 Elektroden) bei Patienten mit OSA erfolgt. Das Verfahren ist somit weniger aufwändig und kostengünstiger als bekannte Verfahren.

Eine entsprechende Vorrichtung zur Durchführung des Verfahrens kann aufgrund der geringen Anzahl von verschiedenen Messsignalen so gestaltet werden, dass sie für die Testperson wenig störend ist. Dies ist insbesondere bei Messungen während des Schlafens von Vorteil, da der Schlaf aufgrund der Messvorrichtung zur Erfassung von Messignalen nicht oder deutlich weniger als bei bisher bekannten Vorrichtungen beeinträchtigt wird. Des Weiteren werden mit Hilfe der Erfindung sowohl der RDI als auch der ESS vollautomatisiert ermittelt, so dass ein objektives Verfahren zur Bestimmung/ Vorhersage der Tagesschläfrigkeit vorliegt.

Bevorzugte Ausführungsformen der Erfindung werden mit Hilfe der beigefügten Zeichnungen näher beschrieben, in denen zeigen:

Fig. 1    eine Übersicht über den Ablauf der Datenerfassung und der Datenanalyse.

Fig. 2    die MI-Unterschiede in Theta-Gamma-FCKW

Fig. 3    die MI-Unterschiede in Delta-alpha-FCKW

Fig. 4    die SVM-Klassifikation verschiedener Schlafstadien

Fig. 5    die SVM-Vorhersage von RDI und ESS

Fig. 6    die Korrelation klinischer Parameter mit CFC-Messungen

Fig. 7    die nachträgliche Verteilung der untersuchten Gruppen und

Fig. 8    eine Messvorrichtung zur Erfassung der EEG-Signale C3 C4

Fig. 9    eine erste alternative Ausführungsform einer Messvorrichtung zur Erfassung der EEG-Signale C3 C4 und

Fig. 10   eine zweite alternative Ausführungsform einer Messvorrichtung zur Erfassung der EEG-Signale C3 C4.

Bei einer mit dem erfindungsgemäßen Verfahren durchgeführten Studie wurden insgesamt 170 Teilnehmer einbezogen. Die Patienten wurden in Haupt- und Validierungsdatensatz mit 86 Teilnehmern im Hauptdatensatz unterteilt: 22 Frauen, Altersgruppe: 27 - 84 Jahre, 44 Probanden mit mittelschwerer oder schwerer OSA und 84 Teilnehmer im Validierungsdatensatz: 28 Frauen, Altersgruppe 35 - 75 Jahre, 42 Probanden mit mittelschwerer oder schwerer OSA. Die für die Analyse verwendeten Daten wurden retrospektiv bewertet. Aufeinanderfolgende Datensätze von Patienten nach Anwendung der unten genannten Ein- und Ausschlusskriterien wurden zur Analyse einbezogen. Daher wurde ein retrospektives unrandomisiertes Fall-Kontroll-Studiendesign verwendet. Alle Patienten hatten zunächst die Ambulanz des Schlafmedizinischen Zentrums eines akademischen medizinischen Zentrums wegen Beschwerden über Schnarchen und / oder Tagesschläfrigkeit besucht. Bei allen Teilnehmern wurde die Diagnose von OSA zunächst anhand der in dieser Studie verwendeten PSG-Aufzeichnungen gestellt. Daher waren alle Teilnehmer therapieunerfahren und hatten keine positive Atemwegsdrucktherapie bzw. Operation der oberen Atemwege oder Therapie mit irgendwelchen Unterkiefervorschubgeräten, die zuvor verwendet wurden.

Die Ein- und Ausschlusskriterien für die Teilnehmer basierten auf Bedingungen, die die Entstehung und den Grad der beobachteten OSA und/oder der EEG-Aufzeichnungen beeinflusst haben könnten. Eingeschlossen waren die Daten von erwachsenen Patienten (im Alter > 18 Jahren), die in der Ambulanz Beschwerden über Schnarchen und/oder Tagesschläfrigkeit hatten, keine vorherige Therapie für schlafbezogene Atmungsstörungen hatten und sich in unserem Schlafmedizinzentrum einer nächtlichen Polysomnographie unterzogen hatten. Teilnehmer mit neurodegenerativen (z. B. Parkinson-Krankheit) oder neuroinflammatorischen (z. B. Multiple Sklerose) Erkrankungen, Schlaganfall in der Anamnese, Herzinsuffizienz basierend auf der New York Heart Association (NYHA) - Stadium 3 oder 4, chronisch obstruktive Lungenerkrankung (COPD), alle psychiatrischen Störungen wurden von der Studie ausgeschlossen. Darüber hinaus wurden Personen mit regelmäßiger Anwendung von Beruhigungsmitteln, Benzodiazepinen, Serotonin-Aufnahmehemmern oder anderen psychotropen Medikamenten, bösartigen Erkrankungen jeglicher Art, Strahlentherapie einer anatomischen Schädel- oder Halsregion, Operationen an intrakraniellen Strukturen oder Operationen zur Therapie schlafbezogener Atmungsstörungen (entweder Schnarchen oder OSA) weiter von der Studie ausgeschlossen. Patientenberichtete Ergebnisse bei übermäßiger Tagesschläfrigkeit (EDS) wurden unter Verwendung der Epworth Sleepiness Scale (ESS) erfasst.

Für die Analyse wurden Ganznacht-Polysomnographie-Aufnahmen (PSG) von 170 gründlich getesteten Personen verwendet, die die Studienzugangskriterien erfüllten. Alle Teilnehmer wurden über Nacht einer Polysomnographie (PSG) mit Aufzeichnungen des Elektroenzephalogramms, des Elektrookulogramms, eines submentalen und prätibialen Elektromyogramms und des Elektrokardiogramms unterzogen. Die Polysomnographie (PSG) wurde nach den aktuellen AASM-Standards (American Academy Sleep Medicine, Inc.) aufgezeichnet, um die Art und den Grad der schlafbezogenen Atmungsstörungen zu bestimmen. Der nasale Luftstrom wurde durch Messung des Aufpralldrucks durch einen Nasensensor dargestellt, in dem Druckschwankungen des Atemluftstroms bestimmt wurden. Thorax- und Bauchexkursionen, Oxyhämoglobinsättigung (Pulsoximeter) und Körperposition wurden gleichzeitig aufgezeichnet. Das Schnarchen wurde mit einem vor dem Kehlkopf befestigten Mikrofon aufgenommen. PSG-Aufnahmen wurden mit einem kommerziell verfügbaren PSG-Messsystem für alle Patienten durchgeführt. Alle EEG-Aufnahmen stammten von C3- und C4-Elektroden mit einer Abtastrate von 200 Hz. Die Patienten wurden in Haupt- und Validierungsdatensatz mit 86 Teilnehmern im Hauptdatensatz unterteilt: 22 Frauen, Altersgruppe: 27 - 84 Jahre, 44 Probanden mit mittelschwerer oder schwerer OSA und 84 im Validierungsdatensatz: 28 Frauen, Altersgruppe 35 - 75 Jahre, 42 Probanden mit mittelschwerer oder schwerer OSA. Die Aufzeichnungsparameter (EEG-Bandpassfilter (0,05-200) Hz, Abtastrate und EEG-Kanäle C2-M1 (linkes Mastoid) und C4-M2 (rechtes Mastoid)) waren in beiden Datensätzen identisch. Alle Polysomnographie-Aufnahmen (PSG) wurden in einer standardisierten Einstellung zwischen 22.00 Uhr und 6.00 Uhr für jeden Patienten durchgeführt.

Die Schlafphasen wurden visuell (manuell) gemäß den Richtlinien der American Academy of Sleep Medicine, Inc. bewertet. Schlafbezogene Atemwegsereignisse wurden visuell (manuell) nach den aktualisierten Richtlinien der American Academy of Sleep Medicine bewertet. Apnoe wurde festgestellt, wenn die maximale Signalauslenkung um $\geq$ 90% gegenüber der Basisline vor dem Ereignis für $\geq$ 10 Sekunden zurückging. In ähnlicher Weise wurde Hypopnoe festgestellt, wenn die maximalen Signalauslenkung für $\geq$ 10 Sekunden um $\geq$ 30% gegenüber der Basisline vor dem Ereignis in Verbindung mit entweder $\geq$ 3% arterieller Sauerstoffentsättigung oder einer kortikalen Erregung sanken. Die weitere Klassifizierung in obstruktive, zentrale oder gemischte respiratorische Apnoe-Ereignisse basierte auf der gleichzeitigen Bewertung des nasalen Luftstroms und der thorakalen und abdominalen Auslenkung.

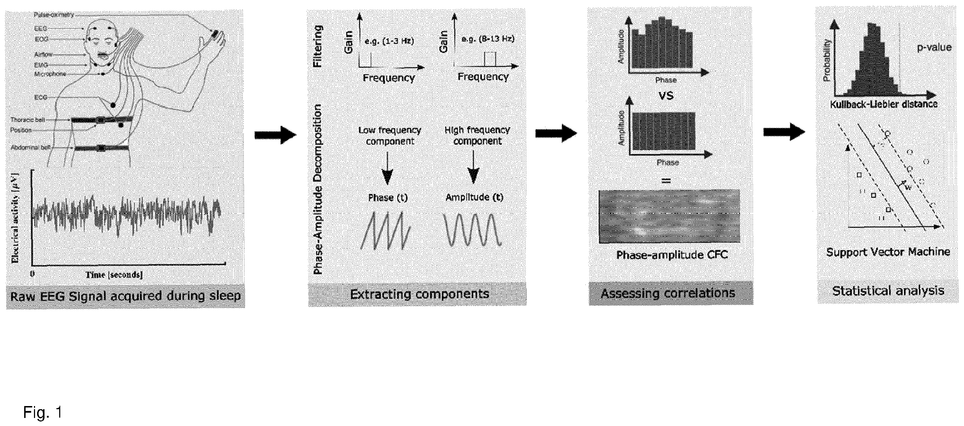

Zur Vorverarbeitung der Daten wurden EEG-Rohdaten tiefpassgefiltert (Butterworth-Filter vierter Ordnung; Grenzfrequenz: 100 Hz), um Aliasing zu vermeiden, gefolgt von Hochpassfilterung bei 0,5 Hz. Für die Artefaktentfernung wurden die Daten unabhängigen Komponentenanalysen (FastICA) unterzogen, um künstliche Komponenten im Zusammenhang mit Muskel-, Augenblinzel-, Augenbewegungs- und Liniengeräuschartefakten zu entfernen. Die vorverarbeiteten Daten wurden dann in sechs verschiedene Frequenzbänder aufgeteilt, nämlich sehr niedrige Frequenzen (VLF, 0,1-1 Hz), Delta (1-3 Hz), Theta (4-7 Hz), Alpha (8-13 Hz), Beta (14-30 Hz) und Gamma (31-100 Hz) für beide Elektroden. Eine Übersicht über den Datenerfassungs- und Analysevorgang ist in Fig. 1 dargestellt.

Die Phase-zu-Amplituden-Kreuzfrequenzkopplung (PACFC) zeigt die Modulation der Hochfrequenzleistung durch die Niederfrequenzphase an. Dieser CFC - Modulationsindex (MI) wird dann verwendet, um die Phasen-Amplituden-Zuordnung zwischen phasenmodulierenden Frequenzbändern (für z.B. Delta) und amplitudenmodulierten Frequenzbändern (für z.B. Alpha) zu identifizieren. Zur Berechnung der CFC - MI wurden folgende Schritte durchgeführt. Zuerst wurde das erhaltene EEG-Signal in zwei Frequenzbereiche gefiltert, nämlich Delta und Alpha. Nach der Filterung wurde die Hilbert-Transformation sowohl auf die gefilterten Zeitreihen angewendet, um die Phase einer Zeitreihe als auch die Amplitudenumhüllung der anderen zu erhalten. Diese kombinierte Zeitreihe hat dann die Information in jeder Phase von Delta-Oszillationen zur Amplitude des Alpha-Rhythmus. Der mögliche Phasenbereich von -180° bis +180° wurde dann in 20 Einheiten (N) mit jeweils 18° unterteilt und der Kullback-Leibler-Abstand (KL) nach folgender Formel berechnet:

$$D_{KL}(P, Q) = \sum_{j=1}^{N} P(j) log\left[\frac{P(j)}{Q(j)}\right]$$

Dabei ist Oder KL-Abstand einer diskreten Verteilung *P* aus einer Verteilung Q. Der KL-Abstand hat die Eigenschaft, immer größer als Null zu sein, d. h. $D_{KL}$ (P,Q) $\geq 0$, es sei denn, die P- und Q-Verteilungen sind gleich, d. h. $D_{KL}$ (P,Q ) = 0, wenn *P* = *Q* und ähnelt der Definition der Shannon-Entropie, die wie folgt angegeben ist:

$$H(P) = -\sum_{j=1}^{N} P(j)log[P(j)]$$

In Bezug auf die Shannon-Entropie kann der KL-Abstand daher verwendet werden, um die Abweichung zwischen der Verteilung der Daten und der gleichmäßigen Verteilung (*U*) wie folgt zu bestimmen:

$$D_{KL}(P,U) = log(N) - H(P)$$

Schließlich wurde die CFC - MI für alle Einheiten berechnet, die Verteilung der mittleren Amplitude ist über alle Einheiten gleichmäßig, was keine Zuordnung zwischen Phase und Amplitude anzeigt. Der Modulationsindex (MI) könnte also wie folgt berechnet werden:

$$MI = \frac{D_{KL}(P,U)}{log(N)}$$

Wenn also die mittlere Amplitude gleichmäßig über die Phasen verteilt ist, wäre der MI Null und wäre maximal, wenn eine Dirac-Delta in der Verteilung der Phasen erhalten wird. Die Kopplung für die Frequenzbänder Theta-gamma und Delta-alpha wurde zwischen der Amplitude der höherfrequenten Signale und der Phase niederfrequenter Signale durch Korrelation geschätzt. Die CFC wurde mit einer Fensterlänge von 5 Sekunden mit einer Überlappung von 50% geschätzt.

Um die Bedeutung dieser CFC-Modulationsindizes zu untersuchen, wurde ein SVM-Algorithmus (Support Vector Machine) verwendet, um verschiedene Schlafstadien basierend auf CFC-MI-Werten aus beiden Frequenzbändern zu klassifizieren. SVM ist ein leistungsfähiges Werkzeug für die nichtlineare Klassifizierung zwischen zwei Datensätzen, das nach einem optimal trennenden Schwellenwert zwischen den beiden Datensätzen sucht, indem es die Marge zwischen den nächstgelegenen Punkten der Klassen maximiert. Hier wurde der Polynomfunktionskern für diese Projektion aufgrund seiner guten Leistung, sowie die Rastersuche (min = 1; max = 10) verwendet, um die wenigen optimalen Eingabeparameter und Gamma (0,25) zu finden. Die Auswahl wurde durch eine 10-fache Kreuzvalidierung überprüft, indem 75% der Daten für das Training und 25% für Tests verwendet wurden. Um die Wirksamkeit dieser CFC-Modulationsindizes für den klinischen Gebrauch zu validieren, wurde die SVM-Analyse weiter angewendet, um die klinischen Scores (RDI und ESS) vorherzusagen, die in den diagnostischen Kriterien für OSA-Patienten verwendet werden. Hier wurde eine Support Vector Regressor (SVR)-Analyse durchgeführt, die eine auf maschinellem Lernen basierende multiple Regressionsmethode darstellt, die die beobachteten und trainierten Werte zuordnen und die Vorhersagegenauigkeit darstellen konnte. Um den Schwellenwert für die Vorhersagegenauigkeit zu erhalten, wurde ein Ansatz entwickelt, der auf der statistischen Inferenz basiert, die aus dem Bayesschen glaubwürdigen Intervall gewonnen wurde. Der 75%-Schwellenwert könnte die posteriore Verteilung vom 95%-Bayes'schen glaubwürdigen Intervall unterscheiden (was auf die Einbeziehung von 95 % der Datenpunkte hinweist). Hier wurden die posteriore Verteilung und das glaubwürdige Intervall unter Berücksichtigung aller Modulationsindizes aus allen Schlafstadien beider Gruppen und des höchsten Dichteintervalls mit 95% (Bereich: 0,32 - 0,89) der Verteilung erhalten. Daher wurde die Vorhersagegenauigkeit von über (75%), die nach der 10-fachen Kreuzvalidierung erzielt wurde, als ziemlich signifikantes Ergebnis angesehen.

Um sicherzustellen, dass es in den Ergebnissen der Studie keine Auswirkungen anderer Variablen als der unabhängigen Variablen gibt, wurden wissenschaftliche Kontrollen durchgeführt. Es wurde überprüft, ob der geschätzte PACFC unabhängig von den Erregungen (arousals) und periodischen Gliedmaßenbewegungen dieser Patienten ist. Dazu wurde der Arousal-Index und der periodische Beinbewegungsindex (PLM) für jeden Patienten sowie die Pearson-Korrelation zwischen dem PACFC in jeder Schlafphase und diesen beiden Indizes geschätzt. Da frühere Studien einen signifikanten Zusammenhang zwischen Herzfrequenzvariabilität und klinischen OSA-Scores gezeigt haben, wurde überprüft, ob der PACFC durch die Aktivität des autonomen Nervensystems beeinflusst wird. Dazu wurde die Herzfrequenzvariabilität (HRV) für jeden Patienten separat geschätzt und die Pearson-Korrelation mit dem PACFC in jeder Schlafphase geschätzt. Die HRV wurde unter Verwendung der Standardabweichung von normal-normalen Intervallen berechnet; eine Technik, die an anderer Stelle beschrieben wird. Weiterhin wurde die Aussagekraft der in der Studie verwendeten Stichprobengröße bestimmt. Dazu wurde eine post-hoc Bayes'sche posteriore Verteilungsanalyse für den MI-Index der N1-Schlafphase zwischen den beiden Gruppen abgeschätzt.

Von den 86 analysierten Patienten wurde bei 42 Patienten ein Atemstörungsindex (RDI) ≤ 15 pro Stunde diagnostiziert (4 mit RDI < 5 pro Stunde und 38 mit RDI zwischen 5 und 15 pro Stunde) und bei 44 Patienten wurde eine klinisch signifikante OSA diagnostiziert (30 Patienten mit RDI zwischen 15 und 30 pro Stunde und 14 Patienten mit RDI > 30 pro Stunde). Diese beiden Gruppen unterschieden sich nicht signifikant in Bezug auf Alter und Geschlecht (p > 0,05). Die demografischen Details zusammen mit den erhaltenen klinischen Maßnahmen sind in Tabelle 1 dargestellt. Die statistische Analyse, die für Cross-Frequency Coupling (CFC)-Parameter durchgeführt wurde, und ihre Verknüpfung mit klinischen Maßnahmen, die aus diesen beiden Gruppen erhalten wurden, ergaben signifikante Ergebnisse, wie unten beschrieben.

**Tabelle 1.** Demografische Details aller in die Studie einbezogenen Teilnehmer. Hier RDI: Index der Atemstörungen; ESS: Epworth Schläfrigkeitsskala.

| Datensatz | Gruppe | N | Alter (Jahre) | Geschlecht | FEI (pro Stunde) | ESS | T-Test p-Werte |
|---|---|---|---|---|---|---|---|
| Hauptgruppe | FEI ≤ 15 | 42 | 55,67 ± 10,22 | F = 19 <br> M = 23 | 9.30 ± 3.26 | 10.90 ± 4.53 | Alter: 0.746 <br> Geschlecht: 0.085 |
| | FEI > 15 | 44 | 56,52 ± 13,91 | F = 12 <br> M = 32 | 27.96 ± 12.47 | 11,59 ± 4,45 | RDI: < 0,001 <br> ESS: 0,480 |
| Validierunggruppe | FEI ≤ 15 | 42 | 52,79 ± 9,71 | F = 17 <br> M = 25 | 11.04 ± 2.97 | 9,86 ± 4,90 | Alter: 0.119 <br> Geschlecht: 0.168 |
| | FEI > 15 | 42 | 56,0 ± 9,02 | F = 11 <br> M = 31 | 49,48 ± 19,67 | 10.14 ± 5.47 | RDI: < 0,001 <br> ESS: 0,817 |

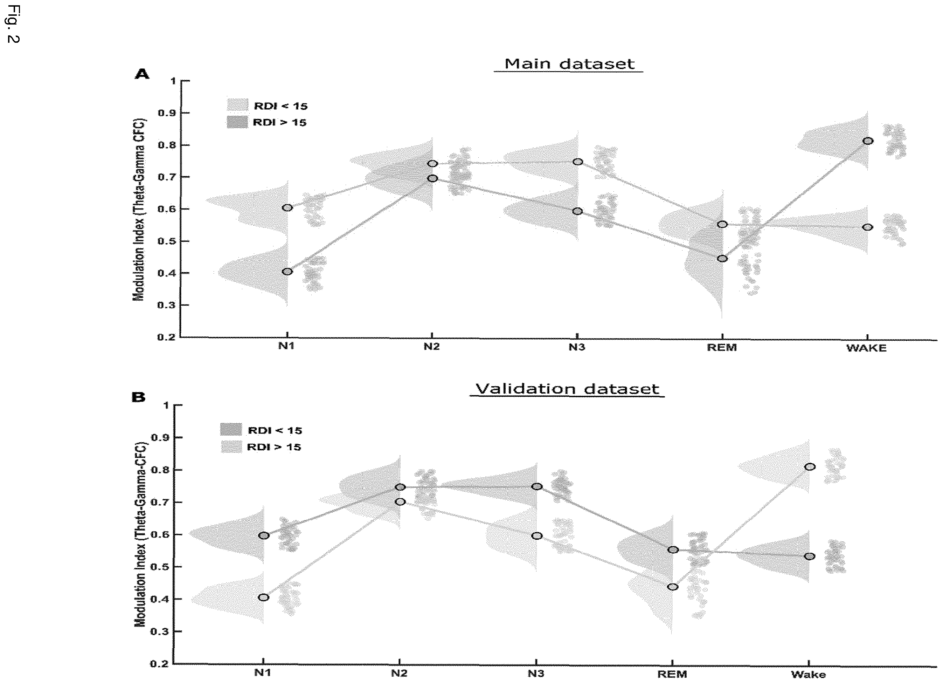

Der CFC-Modulationsindex (MI) in den Theta-gamma-Frequenzbändern war signifikant reduziert (p < 0,001) in allen Schlafstadien bei Patienten mit klinisch signifikanter, d.h. mittelschwerer oder schwerer OSA (RDI > 15/h), wie in Fig. 2A dargestellt. Der Theta-Gamma-Modulationsindex war während der NREM-Stadien N2 und N3 höher als in den N1- und REM-Schlafstadien für beide Gruppen. Der Unterschied in den MI-Werten zwischen den beiden Gruppen war während N1 am höchsten, war während N2 reduziert, nahm aber während der N3- und REM-Schlafphasen wieder zu.

Eine Tabelle, die alle diese Werte darstellt, wird als ergänzende Tabelle 1 bereitgestellt.

Fig. 2 zeigt die MI-Unterschiede in Theta-Gamma-FCKW, wobei das Regenwolkendiagramm in Fig. 2a deutlich zeigt, dass Patienten der RDI-> 15/h-Gruppe in allen Schlafphasen (NREM und REM) einen signifikant niedrigeren Theta-Gamma-CFC-Modulationsindex aufweisen als der der RDI-Gruppe ≤ 15/h, wobei sowohl in der Erst- als auch in der Validierungspatientengruppe genau das gleiche Muster gefunden wurde (Fig. 2b).

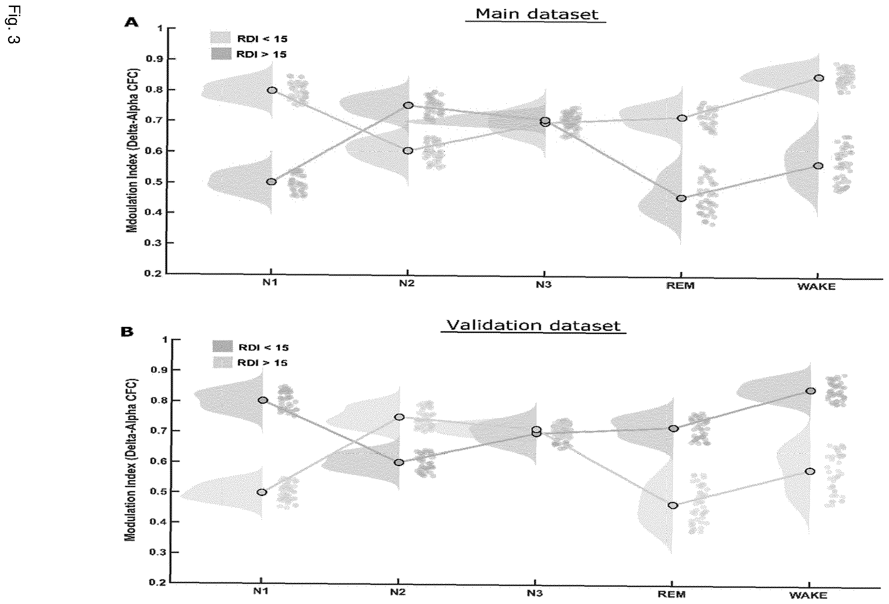

Der CFC-MI an den Delta-alpha-Frequenzbändern war jedoch nur während REM und N1 signifikant reduziert (p < 0,001), nicht jedoch im N3-Schlafstadium bei Patienten mit klinisch signifikanter OSA im Vergleich zu Patienten mit leichter oder keiner OSA (RDI ≤ 15/h), wie in Fig. 3A dargestellt. Darüber hinaus war der CFC - MI im NREM-Schlafstadium N2 bei Patienten mit signifikanter OSA (d.h. RDI > 15/h) höher als bei Patienten ohne (RDI ≤ 15/h). (Siehe Tabelle 1).

Fig. 3 zeigt die MI-Unterschiede in Delta-alpha-FCKW, wobei das Regenwolkendiagramm in Fig. 3a zeigt, dass Patienten der RDI-> 15/h-Gruppe einen signifikant niedrigeren Delta-Alpha-CFC-Modulationsindex als in den REM- und N1-Schlafstadien und einen signifikant höheren MI im N2-Schlafstadium aufweisen als Patienten der RDI-≤ 15/h-Gruppe. Der Delta - Alpha CFC Modulationsindex im NREM-3 (N3) Schlafstadium ist in den beiden Patientengruppen nahezu identisch. Bemerkenswert ist, dass sowohl in den Anfangs- als auch in den Validierungsdatensätzen genau das gleiche Muster wiedergefunden wurde (Fig. 3b).

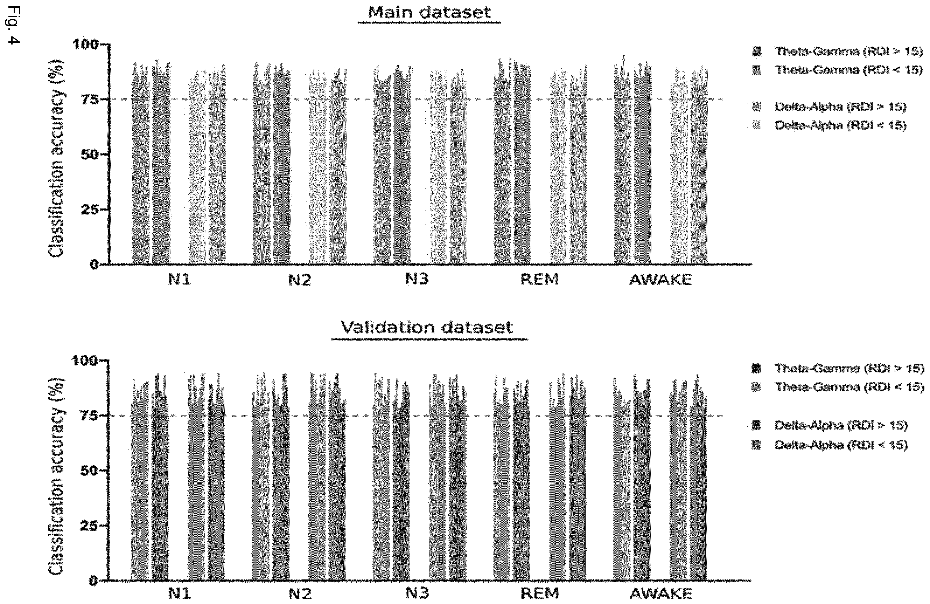

Die SVM-Analyse (Support Vector Machine) zeigte eine signifikante Klassifizierung aller vier Schlafphasen und des Wachstadiums unter Verwendung von CFC-Modulationsindizes getrennt von den Theta-Gamma- und Delta-Alpha-Frequenzbändern. Die gesamte Klassifikationsgenauigkeit war höher als 80% und erreichte bis zu 94% für die Klassifizierung des Wachstadiums unter Verwendung von Theta-gamma CFC-MI, wie in Fig. 4 gezeigt.

Fig. 4 zeigt die SVM-Klassifikation verschiedener Schlafstadien. Das Balkendiagramm zeigt die Klassifizierungsgenauigkeit der Supportvektormaschine (SVM) verschiedener Schlafphasen unter Verwendung von Theta-Gamma- und Delta-Alpha-Kreuzfrequenzkopplungsmodulationsindizes (CFC). Die Gruppe von 10 Balken in jedem Satz stellt die Genauigkeit dar, die für die 10-fache Kreuzvalidierung erhalten wird. Eine gepunktete Linie wird mit einer Genauigkeit von 75 % angezeigt, um den signifikanten Klassifizierungsgrad zu betonen, der für alle in der Studie verwendeten CFC-Metriken erhalten wurde. Alle Genauigkeitswerte sind in der ergänzenden Tabelle 1 dargestellt.

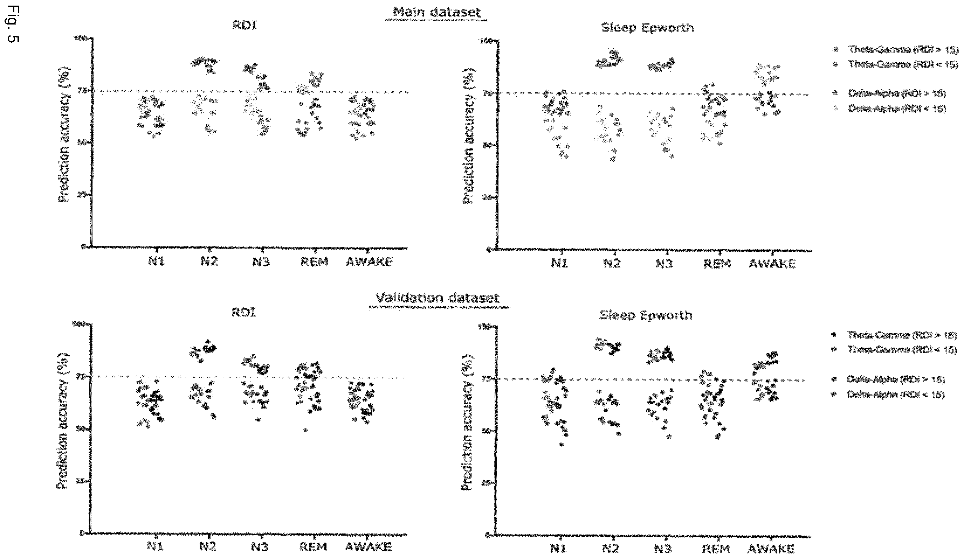

Darüber hinaus konnte mit Hilfe von SVM die RDI und die Epworth-Schläfrigkeitsskala (ESS) in verschiedenen Schlafstadien mit signifikanter Genauigkeit (mehr als 75 %) unter Verwendung des CFC-MI in beiden Frequenzbänderpaaren vorhergesagt werden. Der Theta-gamma-CFC war in der Lage, die RDI und ESS in NREM-Schlafstadien (N2 und N3) signifikant vorherzusagen. Delta-alpha-CFC im REM-Schlafstadium war in der Lage, RDI signifikant vorherzusagen, und Delta-alpha-CFC in Wachstadien war in der Lage, ESS signifikant vorherzusagen. Die Details aller Vorhersagen sind in Fig. 5 dargestellt.

Fig. 5 zeigt die Vorhersage von RDI und ESS. Das Streudiagramm zeigt die Vorhersagegenauigkeit der Supportvektormaschine (SVM) des Atemnotindex (RDI) und der Epworth Sleepiness Scale (ESS) unter Verwendung der Modulationsindizes aus der theta-gamma und delta-alpha Cross Frequency Coupling (CFC). Die Gruppe von 10 Punkten in jedem Satz stellt die Genauigkeit dar, die für die 10-fache Kreuzvalidierung erhalten wird. Eine Genauigkeit von über 75% wurde für signifikant erhalten; dargestellt durch eine gepunktete Linie im Diagramm.

Unter den 84 Patienten, die aus diesem Datensatz analysiert wurden, wurde bei 42 Patienten ein Atemnotindex (RDI) $\leq$ 15 pro Stunde diagnostiziert und bei 42 Patienten wurde eine klinisch signifikante OSA diagnostiziert (3 Patienten mit RDI zwischen 15 und 30 pro Stunde und 39 Patienten mit RDI > 30 pro Stunde). Auch bei diesem Datensatz unterschieden sich die beiden Gruppen hinsichtlich Alter und Geschlecht nicht signifikant (p > 0,05). Die demografischen Details sind in Tabelle 1 dargestellt.

Die für diesen Datensatz durchgeführte statistische Analyse ergab recht ähnliche Ergebnisse wie der erste (Haupt-) Datensatz und bestätigte daher die meisten Ergebnisse. Die CFC-MI an den Theta-Gamma-Frequenzbändern waren auch bei klinisch signifikanten OSA-Patienten (RDI > 15/h) reduziert, ähnlich wie in den Hauptbefunden, die eine höhere Modulation auch während der NREM-N2- und N3-Schlafphasen zeigten (Fig. 2B). Ebenso wurde die CFC-MI in Delta-alpha-Frequenzbändern auch nur während REM und N1 signifikant reduziert, nicht jedoch in den Schlafstadien N2 und N3 bei Patienten mit klinisch signifikanter OSA, wie im Hauptdatensatz zu finden ist (Fig. 3B).

Die SVM-Analyse zur Klassifizierung von Schlafstadien unter Verwendung von CFC-Modulationsindizes für Theta-Gamma- und Delta-Alpha-Frequenzbänder zeigte replizierbare Ergebnisse unter Verwendung des Validierungsdatensatzes mit einer Klassifizierungsgenauigkeit von über 80%, wie in Fig. 4 dargestellt.

In ähnlicher Weise war der Validierungsdatensatz außerdem in der Lage, die Vorhersageergebnisse für klinische Parameter - RDI und Epworth Sleepiness Score (ESS) - mit einer Genauigkeit von mehr als 75% zu replizieren, wobei die gleichen CFC-Modulationsindizes wie im Hauptdatensatz verwendet wurden, wie in Fig. 5 dargestellt.

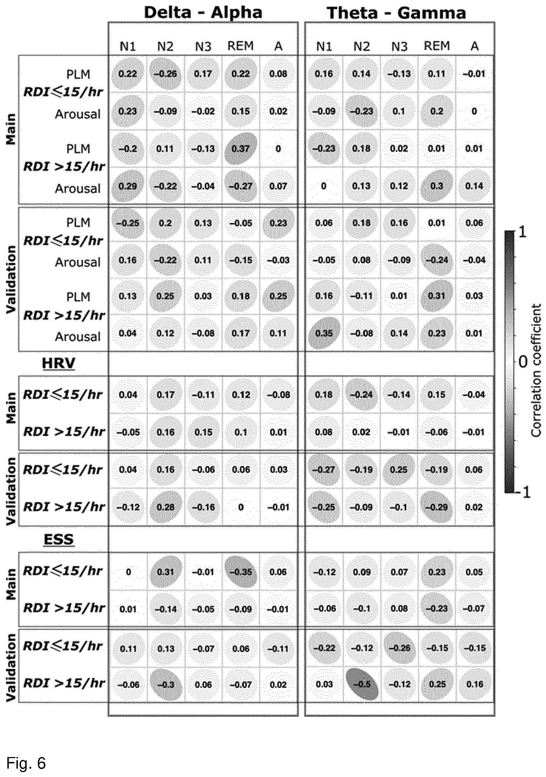

Die Korrelation zwischen dem Epworth-Schläfrigkeitswert (ESS) und PACFC war jedoch auch in keinem Schlafstadium signifikant (Fig. 6).

Es wurde keine signifikante Korrelation (alle p > 0,05) zwischen den Arousal- und PLM-Indizes zum PACFC in jeder Schlafphase gefunden (Fig. 6). 2) Darüber hinaus wurde keine signifikante Korrelation zwischen der Herzfrequenzvariabilität und PACFC in irgendeinem Schlafstadium, die keinen Einfluss des autonomen Nierensystems auf PACFC zeigte, gefunden (Fig. 6).

In Fig. 6 ist die Korrelation klinischer Parameter mit CFC-Messungen dargestellt. Fig. 6a zeigt die Korrelationskoeffizienten zwischen Arousal- und periodischen Beinbewegungsindizes (PLM) zur Phasenamplituden-Kreuzfrequenzkopplung (PACFC) für jede Schlafphase separat. Sowohl für Delta-alpha als auch für Theta-gamma sind PACFC durch die Spalten getrennt. Fig. 6b zeigt die Korrelationskoeffizienten zwischen der Herzfrequenzvariabilität (HRV) und dem PACFC in den verschiedenen Schlafstadien durch Spalten getrennt. Fig. 6c zeigt die Korrelationskoeffizienten zwischen dem Epworth-Schläfrigkeitswert (ESS) und dem PACFC in verschiedenen Schlafstadien. Die Korrelation für die Haupt- und die Validierungsgruppe wird in jeder Zeile separat angezeigt. Die r-Werte werden für die Korrelation angezeigt. Alle Korrelationen waren nicht signifikant (p> 0,05).

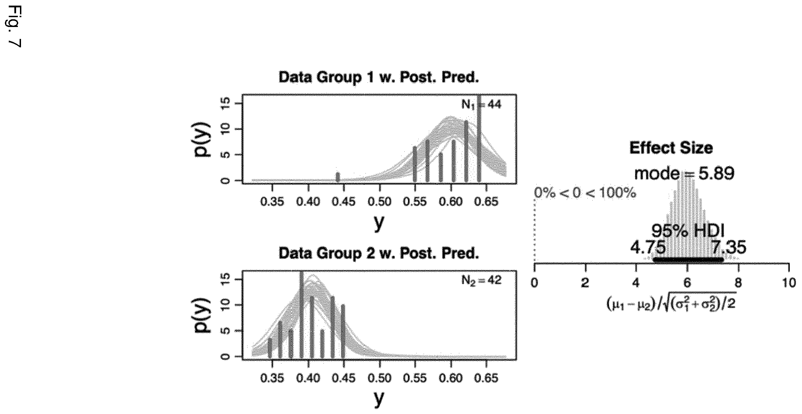

Die Bayessche posteriore Verteilung zeigte, dass das 95%ige High Density Intervall (HDI) innerhalb des erhaltenen Effekts in den analysierten Daten liegt (Fig. 7), was auf eine ausreichende Stichprobengröße für das primäre Ergebnis in dieser Studie hinweist.

Fig. 7 zeigt die nachträgliche Verteilung der untersuchten Gruppen. Das Diagramm auf der rechten Seite zeigt das Verteilungshistogramm der Effektgröße, das das 95% High Density Intervall (HDI) anzeigt, das sich in den analysierten Daten befindet. Dies deutet auf eine ausreichende Stichprobengröße der eingeschlossenen Probanden basierend auf dem primären Ergebnis hin (d. h. Phasenamplituden-Kreuzfrequenzkopplung für Theta - Gamma im N1-Schlafstadium). Die Diagramme auf der linken Seite zeigen die Wahrscheinlichkeitsverteilung mit überlagerter nachträglicher prädiktiver Verteilung der Rohdaten für jede Datenstichprobe.

Es wurde eine signifikante Reduktion des Theta-Gamma-Modulationsindex (MI) in den zentralen sensomotorischen kortikalen Regionen bei Patienten mit mittelschwerer oder schwerer OSA im Vergleich zu Patienten mit leichter OSA oder gesunden Personen gefunden. Die MI-Reduktion während des Schlafes war frequenzbandspezifisch; Es beinhaltete die Theta-Gamma-Konnektivität während aller Schlafphasen, während der Delta-Alpha-Konnektivität nur während REM und N1. Daher wurde während REM und N1 eine globale Reduktion der Modulation (sowohl Theta-gamma als auch Delta-alpha) beobachtet.

Darüber hinaus waren die MI-Unterschiede zwischen den Stadien so ausgeprägt, dass in beiden Datensätzen eine Schlafstadienklassifizierung anhand von MI-Werten in beiden Patientengruppen erreicht wurde. Darüber hinaus sagte Theta-gamma MI während N2 und N3 sehr zuverlässig sowohl RDI als auch ESS voraus und Delta-alpha MI sagte RDI während REM sehr zuverlässig voraus.

Diese neuartigen Ergebnisse, die die funktionelle Trennung zwischen Theta- und GammaAktivität im kortikalen sensomotorischen Bereich während aller Schlafstadien bei OSA-Patienten zeigen, haben pathophysiologische und klinische Implikationen, die weiter diskutiert werden müssen.

Theta-gamma PACFC wurde mit motorischen, sensorischen und kognitiven Prozessen in Verbindung gebracht. Bei Personen mit RDI ≤ 15/h besteht sowohl in den N2- als auch in den N3-Stadien eine sehr starke Kopplung zwischen Theta- und Gamma-Oszillationen. Bei mittelschwerer und schwerer OSA nimmt der Modulationsindex während N3 recht deutlich ab, während er in N2 in viel geringerem Maße abnimmt. Während kurzer Wachphasen zwischen den Schlafphasen stieg der MI bei Patienten mit mittelschwerer / schwerer (d. h. signifikanter) OSA im Vergleich zu Patienten mit RDI ≤ 15 / h signifikant an. Dies kann eine physiologische (motorische, respiratorische, kognitive) kompensatorische Rolle für kortikale Arousals (Erregungen) und / oder intermittierende Wachphasen widerspiegeln, um einen vorübergehenden Anstieg der zentralen sensomotorischen Konnektivität bei Patienten mit signifikanter OSA zu fördern. Eine synaptische Nettopotenzierung im Zusammenhang mit Wachzuständen kann die Grundlage für eine solche Zunahme der Konnektivität bilden.

Die aufgezeichnete neuronale Aktivität des sensomotorischen Kortex kann entweder primär in diesem anatomischen Bereich erzeugt werden oder ein Epiphänomen der Aktivität anderer subkortikaler / thalamischer oder neuraler Master-Generatoren des Hirnstamms sein. Diese Generatoren fördern die Neuromodulation der Hirnnervenbahnen, was zu dem reduzierten Muskeltonus der oberen Atemwege führt, der mit den Obstruktionen der oberen Atemwege verbunden ist, die während der Atemwegsereignisse im Schlaf bei OSA auftreten.

Bei Patienten mit fokalen epileptischen Anfällen war die Intensität der Theta-gamma-Phasenamplitudenkopplung während des Schlafes die höchste während N3 und die niedrigste während der REM. Bei mittelschwer und stark betroffenen OSA-Patienten war Theta-gamma-PACFC während N2 am höchsten (siehe Fig. 2). Die Kopplung von schnellen und langsamen Schwingungen war während der REM im Vergleich zu N2 und N3 in allen OSA-Patientengruppen stark reduziert; Diese Reduktion war bei Patienten mit signifikanter OSA ausgeprägter. Starke CFC zwischen hochfrequenten und langsamwelligen Schwingungen während des langsamwelligen Schlafes wurde im Hippocampus anästhesierter Primaten gefunden. Das EEG misst die summierten postsynaptischen Potenziale synchron aktiver Regionen von Kortex und Hippocampus, die sich über Gehirn, Schädel und Kopfhaut ausgebreitet haben. Obwohl sie oft zusammenfallen, ist es nicht notwendig, dass das Abfeuern von Aktionspotentialen mit Schwingungen postsynaptischer Potentiale zusammenhängt. Daher führen die Schwingungen postsynaptischer Potentiale nicht immer zum Abfeuern postsynaptischer Aktionspotentiale. Es ist schwierig, die kortikale von der hippokampalen Ausgabe beim Menschen allein auf der Grundlage von Oberflächen-EEG-Aufzeichnungen zu unterscheiden.

Ein signifikanter Anstieg der Delta-alpha-CFC - MI wird während N2 bei Patienten mit signifikanter OSA im Vergleich zu Patienten mit RDI ≤ 15/h beobachtet. Dieser Befund kann eine kompensatorisch erhöhte Aktivität des sensomotorischen Kortex während des respiratorischen Ereignis-Rich-N2-Stadiums darstellen, um eine bessere motorische Kontrolle der Atmung bei schwerer (RDI > 15/h) betroffenen OSA-Patienten auszuüben.

Delta-Band-Oszillation in Spike und lokalen Feldpotentialen Aktivität im somatosensorischen Whisker-Barrel-Kortex von wachen Mäusen ist phasengebunden an die Atmung. Daher moduliert die Atmungsaktivität direkt die langsame (1-4 Hz) rhythmische neuronale Aktivität im somatosensorischen Whisker-Barrel-Kortex und indirekt die Gammabandleistung durch Phasen-Amplituden-Kopplungsmechanismen in Mäusen. Unsere Ergebnisse liefern vorläufige Hinweise auf eine physiologische Beteiligung von Delta- und Gammabandoszillationen an der Kontrolle der Atmung beim Menschen, insbesondere bei OSA, und sollten weiter validiert werden.

Insbesondere bleibt der Delta-Alpha-CFC-MI während N3 ziemlich stabil, unabhängig vom OSA-Schweregrad. Daher kann die Delta-Alpha-Kopplung an Gehirnkonnektivitätsprozessen beteiligt sein, die während N3 stabil bleiben, oder sie kann auch ein Ersatzmarker für die bekannte Atemstabilität während N3 sein, wenn Apnoen und Hypopnoen viel seltener auftreten.

Angesichts der Tatsache, dass sowohl Theta-Gamma- als auch Delta-Alpha-Modulationsindizes die Schlafstadionklassifizierung nach AASM-Kriterien zuverlässig vorhersagen können, kann vermutet werden, dass ganz unterschiedliche schlafphasenspezifische PACFC-Muster mit den oben genannten Frequenzbändern existieren. Diese unterschiedlichen Muster sind offenbar recht robust, betreffen mindestens die beiden oben genannten (Gamma-Theta, Delta-Alpha) Schwingungskanäle und können weitere oszillierende Kanäle beinhalten. Da ein großer Teil der getesteten Datensätze Patienten mit RDI > 15/h gehört, scheinen diese schlafstadienspezifischen Kopplungsmuster unabhängig vom Grad der begleitenden schlafbedingten Atemnot recht robust zu bleiben.

Die globale (Theta-gamma- und Delta-alpha) Konnektivitätsreduktion, wie sie durch die MI-Reduktion dargestellt wird, während REM in den zentralen sensomotorischen Bereichen bei OSA-Patienten mit RDI>15/h im Vergleich zu Patienten mit RDI ≤ 15/h kann einen Surrogatmarker für die reduzierte zentrale Motorleistung während REM liefern. Diese reduzierte motorische Leistung betrifft wahrscheinlich viele Muskelgruppen und betrifft insbesondere die Muskeln, die die Durchgängigkeit der oberen Atemwege kontrollieren, da sie stark mit der RDI korrelieren. Besonders ausgeprägt ist der MI-Unterschied bei Delta-alpha CFC-MI (Fig. 2). Eine differentielle Modulation von globalen und lokalen Schwingungen während des REM-Schlafes wurde berichtet. Daher kann die multifrequente (globale) MI in den sensomotorischen Bereichen während der REM als Surrogatmarker für die Schwere der OSA-Erkrankung dienen. Zur Stützung dieses Arguments hat eine weitere Analyse (Fig. 4) gezeigt, dass Delta-alpha MI während REM in beiden getesteten Datensätzen sehr zuverlässig die durchschnittliche RDI vorhergesagt hat.

Theta-gamma MI während N2 und N3 und Delta-alpha MI während kurzer Wachphasen aus dem Schlaf erweisen sich als zuverlässige Surrogatmarker für patientenberichtete übermäßige Tagesschläfrigkeit (EDS, excessive daytime sleepiness). Diese Ergebnisse weisen auch auf mögliche schlafbezogene oszillations- und stadionspezifische physiologische Mechanismen hin, die Aufmerksamkeit und Wachsamkeit beim Menschen fördern. Subjektive Bewertungen der Schläfrigkeit haben ausgeprägte Assoziationen mit erhöhter funktioneller Konnektivität in weit verbreiteten Regionen innerhalb des sensomotorischen Netzwerks gezeigt.

Der Phasen-Amplituden-Cross-Frequency Coupling (PACFC)-Modulationsindex war das primäre Endergebnis und wurde in dieser Studie als Prädiktor für die klinischen Variablen getestet. Zu seiner Bedeutung: Räumliche Arbeitsgedächtnisleistung, Aufrechterhaltung des Arbeitsgedächtnisses mit mehreren Elementen, Veränderungen der Wahrnehmungsergebnisse, Lernen, visuelle Aufmerksamkeit und Wahrnehmung sind einige der funktionellen Merkmale, die mit der PACFC-Modulation in Verbindung gebracht wurden. Darüber hinaus wurde auch gezeigt, dass es zur BOLD-Konnektivität (abhängig vom Blutsauerstoffspiegel) beiträgt und Verbindungen zu Gehirnveränderungen aufweist, die bei mehreren neurologischen Erkrankungen wie Epilepsie, Parkinson-Krankheit, Alzheimer-Krankheit, Schizophrenie, Zwangsstörungen (OCD) und leichten kognitiven Beeinträchtigungen (MCI, minimal cognitive impairment) auftreten. Obwohl es genügend Beweise dafür gibt, dass PACFC potenziell ein vielversprechender Ansatz ist, um die Gehirnfunktion und einige ihrer Pathologien mit einem glaubwürdigen physiologischen Mechanismus zu entschlüsseln (die Niederfrequenzphase spiegelt die lokale neuronale Erregbarkeit wider, während hochfrequente Leistungssteigerungen entweder einen allgemeinen Anstieg der synaptischen Aktivität der Bevölkerung oder die selektive Aktivierung eines verbundenen neuronalen Subnetzwerks widerspiegeln). Es gibt noch einige unbeantwortete Fragen zur Entstehung, Kausalität und zum Mechanismus dieser Schwingungen. Die Wahl des Modulationsindex (MI) in dieser Studie basiert auf der Erkenntnis, dass sich MI unter einigen der am häufigsten verwendeten Phasenamplitudenkopplungsmessungen als am robustesten gegen verwirrende Einflüsse von Moderatoren erwiesen hat, einschließlich Datenlänge, Signal-Rausch-Verhältnis und Abtastrate, wenn man sich Nyquist-Frequenzen nähert.

Die oben genannten Beweise können neue Möglichkeiten der Intervention durch pharmakologische oder transkranielle Magnetstimulation (TMS) des sensomotorischen Kortex bei OSA-Patienten eröffnen. TMS während des Schlafes wurde auf die kortikomotorisch-somatotopische Darstellung der Zunge angewendet; Induzierte Zuckungen haben den Luftstrom kurzzeitig verbessert, ohne Arousals (Erregungen) bei OSA-Patienten zu verursachen. Die Wirkung auf andere motorische Bereiche und die neurokognitive Wirkung von TMS wurde jedoch in OSA nicht umfassend untersucht. Der Befund, dass die oben genannten CFC-Modulationsindizes in beiden Frequenzbändern den RDI- und Epworth-Schläfrigkeitsscore (ESS) bei OSA-Patienten signifikant vorhersagen könnten, sollte in größeren Studien weiter validiert werden.

Das Ergebnis, dass 1) Theta-gamma CFC-MI signifikant die RDI und ESS in NREM (N2, N3) vorhergesagt hat, 2) Delta-alpha CFC-MI RDI in REM vorhergesagt hat und 3) Delta-alpha CFC-MI in wachen Zuständen signifikant ESS vorhergesagt hat, deuten darauf hin, dass die CFC-MI Theta-gamma- und Delta-alpha-Metriken ganz unterschiedliche Prozesse in der menschlichen Schlafphysiologie darstellen können. Die Delta-Alpha-Kopplung scheint signifikant zu sein, 1) für die Kontrolle der motorischen Stabilität und Atmung der oberen Atemwege während des REM-Schlafes und 2) für die Kontrolle von Aufmerksamkeits- und Wachsamkeitsprozessen, wie sie von ESS dargestellt werden, während der (kortikalen) Erregung und kurzer Wachphasen zwischen den Schlafphasen. Die Theta-gamma-Phasenamplitudenkopplung scheint sehr signifikant zu sein, 1) für die Kontrolle der Stabilität und Atmung der oberen Atemwege während des NREM N2- und N3-Schlafes und 2) für aufmerksamkeits- und wachsamkeitsbezogene Prozesse (dargestellt durch ESS), die während des N2- und N3-Schlafes auftreten. Diese Ergebnisse deuten darauf hin, dass die kortikalen zentralen sensomotorischen Regionen tatsächlich ein bedeutender Knotenpunkt in den Netzwerken sein können, die den Schlaf und / oder die schlafbezogene Atemaktivität regulieren. Nach der Validierung in größeren Patientenkohorten könnte der Modulationsindex schließlich als zusätzliche Metrik integriert werden, die sowohl die Schwere der Atemnot als auch die Tagesschläfrigkeit bei Patienten mit OSA darstellt.

Die Replikation der Befunde im Validierungsdatensatz unterstützt die Reproduzierbarkeit und Validität der Ergebnisse weiter und weist auf ihre klinische Bedeutung als diagnostische Surrogatmarker hin. Darüber hinaus zeigten die wissenschaftlichen Kontrollergebnisse eindeutig keinen Einfluss der Erregung, der periodischen Beinbewegung sowie des autonomen Nervensystems in der PACFC-Messung.

Es wird vorgeschlagen, dass der Theta-gamma-MI an den sensomotorischen kortikalen Bereichen während N2 und N3 und der Delta-alpha-CFC-MI an den sensomotorischen kortikalen Bereichen während REM als Metrik der Atemnot während des Schlafes beim Menschen und damit als Messgröße des OSA-Schweregrads verwendet werden kann.

Daher sollten weitere Untersuchungen von Theta-Gamma-FCKW während N2 und N3 und Delta-alpha-FCKW während REM durchgeführt werden. Die Berechnung dieser MIs in weiteren kortikalen Bereichen kann zusätzliche Einblicke in die OSA-Pathogenese und - Diagnose liefern. Neurophysiologische und neuroimaging-Untersuchungen zur thalamokortikalen Konnektivität, die auf den vorliegenden Ergebnissen basieren, können Mechanismen übermäßiger Tagesschläfrigkeit weiter aufklären. Darüber hinaus wäre es interessant, die Wirkung etablierter evidenzbasierter Therapien für OSA, wie z.B. der Therapie mit positivem Atemwegsdruck (PAP), auf PACFC zu bewerten.

Die funktionelle Trennung des zentralen kortikalen sensomotorischen Bereichs zwischen Theta- und Gammaaktivität wird in allen Schlafstadien der OSA beobachtet. Eine weitere signifikante Delta-Alpha-sensomotorische Flächentrennung tritt während der REM- und N1-Stadien in OSA auf. Daher ist die sensomotorische Trennung weit verbreitet, zeigt frequenzband- und schlafstufenspezifische Muster und liefert weitere Hinweise auf das Vorhandensein einer zentralen sensomotorischen Dysfunktion bei OSA-Patienten. Der Theta-Gamma-Modulationsindex während N2 und N3 sagte zuverlässig die vom Patienten berichtete Schläfrigkeit voraus. Daher können Modulationsindizes als Surrogat-diagnostische prädiktive Marker für Atemnot während des Schlafes und für von Patienten berichtete übermäßige Tagesschläfrigkeit verwendet werden.

Zusammengefasst ist das beschriebene Verfahren geeignet zur Bestimmung des Schweregrads der obstruktiven Schlafapnoe und der begleitenden Tagesschläfrigkeit wobei das Verfahren folgende Schritte umfasst:

- Erfassen zweier Körperfunktionsdaten, wobei die beiden Körperfunktionsdaten EEG Messsignale einer Elektroenzephalographie an den Elektroenzephalographiestellen C3 und C4 sind,
- Aufteilen der Messignale in Frequenzbänder, wobei die Aufteilung für jede Messstelle getrennt erfolgt,
- Bestimmen von Kreuzfrequenzmodulationsindizes,
- Bestimmen des Respiratory Disturbance Index und der Tagesschläfrigkeit mittels einer Support Vector Machine auf Basis der Kreuzfrequenzmodulationsindizes,

Der Respiratory Disturbance Index und die Tagesschläfrigkeit sind hier ein Maß für den Schweregrad der obstruktiven Schlafapnoe und seiner Begleiterscheinungen.

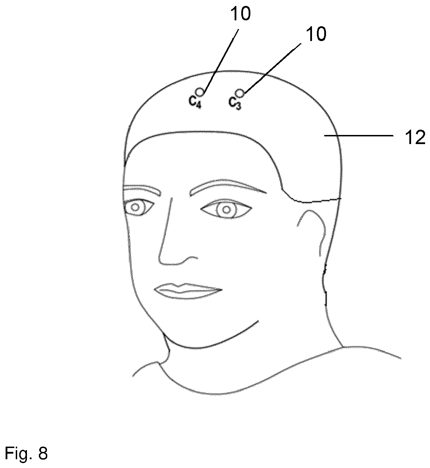

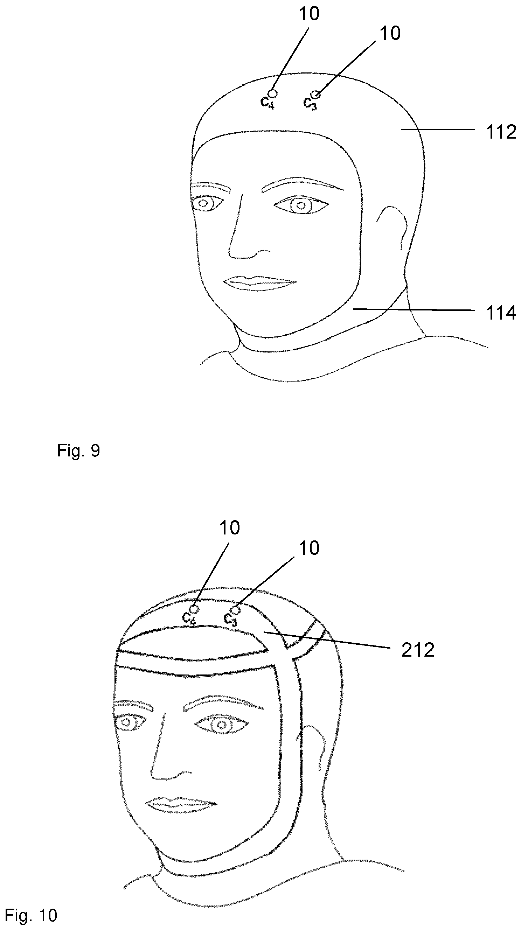

In den Fig. 8 bis 10 sind Ausführungsformen einer Messvorrichtung zur Erfassung der Messignale C3 und C4 gezeigt. Diese Messvorrichtungen sind Teil einer nicht dargestellten Vorrichtung zur Durchführung des beschriebenen Verfahrens. Bei den Messvorrichtungen in den Fig. 8 und 9 handelt es sich um eine Kopfbedeckung, in der zwei Sensoren 10 zur Erfassung der C3 und C4 Messignale eingearbeitet sind. Die Kopfbedeckungen sind hier als Kappe 12 (Fig. 8) oder als Kappe 112 mit Kinnteil 114 (Fig. 9) ausgebildet. Alternativ können als Kopfbedeckung auch miteinander verbundene Bänder 212 vorgesehen sein, die am Kopf befestigt werden und in denen zwei Sensoren 10 zur Erfassung der C3 und C4 Messignale eingearbeitet sind (siehe Fig. 10).

## Patentansprüche

1. Verfahren zur Bestimmung eines Maßes für den Grad einer obstruktiven Schlafapnoe und/oder deren Folgeerscheinung mittels folgender Schritte:

   - Festlegen eines Maßes für den Grad einer obstruktiven Schlafapnoe und/oder deren Folgeerscheinung,
   - Bereitstellung zweier EEG-Messignale einer Elektroenzephalographie an den Elektroenzephalographiestellen eines 10-20 internationalen EEG Systems sind,
   - Aufteilen der EEG- Messignale in Frequenzbänder,
   - Bestimmen wenigstens eines Kreuzfrequenzmodulationsindex mittels Daten aus wenigstens zwei verschiedenen Frequenzbändern,
   - Bestimmen des Maßes für den Grad einer obstruktiven Schlafapnoe und/oder dessen Folgeerscheinung mittels des wenigstens einen Kreuzfrequenzmodulationsindex.

2. Verfahren nach Anspruch 1, **dadurch gekennzeichnet, dass** die EEG- Messignale während des Schlafens im Labor oder Zuhause, ermittelt werden.

3. Verfahren nach einem der vorhergehenden Ansprüche, **dadurch gekennzeichnet, dass** das Maß für den Grad einer obstruktiven Schlafapnoe und/oder dessen Folgeerscheinung, insbesondere der Respiratory Disturbance Index und/oder die Tagesschläfrigkeit, nur auf Basis der beiden EEG Messignale bestimmt wird.

4. Verfahren nach einem der vorhergehenden Ansprüche, **dadurch gekennzeichnet, dass** die Elektroenzephalographiestellen, an denen die Messsignale erfasst werden, die Stellen C3 und C4 sind.

5. Verfahren nach Anspruch, **dadurch gekennzeichnet, dass** die Aufteilung der Messsignale in Frequenzbänder für jede Messstelle getrennt erfolgt.

6. Verfahren nach einem der vorhergehenden Ansprüche, **dadurch gekennzeichnet, dass** der wenigstens eine Kreuzfrequenzmodulationsindex mittels einer Phase-Amplituden Kreuzfrequenzkopplung ermittelt wird.

7. Verfahren nach einem der vorhergehenden Ansprüche, **dadurch gekennzeichnet, dass** die Messsignale in wenigstens zwei der folgenden Frequenzbänder aufgeteilt werden: niedriges Frequenzband von 0,1 bis 1 Hz, Delta-Band (1 bis 3 Hz), Theta-Band (4 bis 7 Hz), Alpha-Band (8-13 Hz), Beta-Band (14 bis 30 Hz) und Gamma-Band (31- 100 Hz).

8. Verfahren nach einem der Ansprüche 6 oder 7, **dadurch gekennzeichnet, dass** zur Ermittlung einer Phase-Amplituden Kreuzfrequenzkopplung die Messsignale in die beiden Frequenzbänder Alpha-Band (8-13 Hz) und Delta-Band (1 bis 3 Hz) aufgeteilt werden, wobei vorzugsweise mittels der Phase-Amplituden-Kreuzfrequenzkopplung aus dem Alpha-Band die Amplituden- Hüllkurve und aus dem Delta-Band die Phase der Messignale bestimmt werden.

9. Verfahren nach einem der Ansprüche 6 bis 8, **dadurch gekennzeichnet, dass** zur Ermittlung einer Phase-Amplituden Kreuzfrequenzkopplung die Messsignale in die beiden Frequenzbänder Theta-Band (4 bis 7 Hz) und Gamma-Band (31- 100 Hz) aufgeteilt werden, wobei vorzugsweise mittels der Phase-Amplituden Kreuzfrequenzkopplung aus dem Gamma-Band die Amplituden- Hüllkurve und aus dem Theta-Band die Phase der Messignale bestimmt werden.

10. Verfahren nach einem der vorhergehenden Ansprüche, **dadurch gekennzeichnet, dass** eine Korrelationsdatenbank mit Korrelationsdaten zwischen dem wenigstens einen Kreuzfrequenzmodulationsindex und dem Maß für den Grad einer obstruktiven Schlafapnoe und/oder dessen Folgeerscheinung bereitgestellt wird und das Maß für den Grad einer obstruktiven Schlafapnoe und/oder dessen Folgeerscheinung der wenigstens mit Hilfe der Daten der Korrelationsdatenbank aus dem wenigstens einen Kreuzfrequenzmodulationsindex bestimmt wird.

11. Verfahren nach einem der vorhergehenden Ansprüche, **dadurch gekennzeichnet, dass** das Maß für den Grad einer obstruktiven Schlafapnoe und/oder dessen Folgeerscheinung insbesondere der Respiratory Disturbance Index und/oder der Tagesschläfrigkeit mittels einer Support Vector Machine auf Basis des wenigstens einen Kreuzfrequenzmodulationsindex bestimmt werden.

12. Verfahren nach einem der vorhergehenden Ansprüche, **dadurch gekennzeichnet, dass** die Messsignale während des Schlafs erfasst werden und der Schlaf in Schlafstadien eingeteilt wird, wobei der wenigstens eine Kreuzfrequenzmodulationsindex in Abhängigkeit von Schlafstadien bestimmt wird.

13. Verfahren nach einem der vorhergehenden Ansprüche, **dadurch gekennzeichnet, dass** alle Verfahrensschritte automatisch durchgeführt werden.

14. Vorrichtung zur Durchführung eines Verfahrens zur Bestimmung eines Maßes für den Grad einer obstruktiven Schlafapnoe und/oder deren Folgeerscheinung, insbesondere Vorrichtung zur Durchführung eines Verfahrens nach einem der vorhergehenden Ansprüche, **dadurch gekennzeichnet, dass** die Vorrichtung eine Kopfbedeckung mit nur zwei Sensoren zur Bestimmung der EEG-Signale umfasst, wobei die Sensoren vorzugsweise so angebracht sind, dass die Messsignale an den Stellen C3 und C4 erfasst werden.

15. Vorrichtung nach Anspruch 14, dass die Kopfbedeckung ein Stirnband, eine Kappe oder eine Mütze ist.

Fig. 1

Fig. 2

Fig. 3

Fig. 4

Fig. 5

18

Fig. 6

Fig. 7

Fig. 8

Fig. 9

Fig. 10

Europäisches Patentamt

European Patent Office

Office européen des brevets

**EUROPÄISCHER RECHERCHENBERICHT**

Nummer der Anmeldung

**EP 23 15 2373**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | KOLEY BIJOY LAXMI ET AL: "Classification of sleep apnea using cross wavelet transform", 2013 IEEE 1ST INTERNATIONAL CONFERENCE ON CONDITION ASSESSMENT TECHNIQUES IN ELECTRICAL SYSTEMS (CATCON), IEEE, 6. Dezember 2013 (2013-12-06), Seiten 275-280, XP032565126, DOI: 10.1109/CATCON.2013.6737512 | 1-6,13 | INV. A61B5/00 A61B5/374 |
| Y | * Zusammenfassung * * Seite 276 - Seite 277 * ----- | 7-12 | |
| Y | DE 10 2020 125743 A1 (UNIV DER JOHANNES GUTENBERG UNIV MAINZ KOERPERSCHAFT DES OEFFENTLICHEN) 7. April 2022 (2022-04-07) * Absätze [0020], [0025], [0034], [0050], [0051] * ----- | 7-12 | |
| X | DE 10 2019 129288 A1 (UNIV DER JOHANNES GUTENBERG UNIV MAINZ [DE]) 6. Mai 2021 (2021-05-06) * Abbildung 1 * ----- | 14,15 | RECHERCHIERTE SACHGEBIETE (IPC) A61B |
| A | VIMALA V ET AL: "An Intelligent Sleep Apnea Classification System Based on EEG Signals", JOURNAL OF MEDICAL SYSTEMS, SPRINGER US, NEW YORK, Bd. 43, Nr. 2, 8. Januar 2019 (2019-01-08) , Seiten 1-9, XP036687856, ISSN: 0148-5598, DOI: 10.1007/S10916-018-1146-8 [gefunden am 2019-01-08] * Seiten 3-6 * ----- | 1-15 | |

Der vorliegende Recherchenbericht wurde für alle Patentansprüche erstellt

| Recherchenort | Abschlußdatum der Recherche | Prüfer |
|---|---|---|
| Den Haag | 5. September 2023 | Worms, Georg |

**ANHANG ZUM EUROPÄISCHEN RECHERCHENBERICHT
ÜBER DIE EUROPÄISCHE PATENTANMELDUNG NR.**

EP 23 15 2373

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-09-2023

| Im Recherchenbericht angeführtes Patentdokument | Datum der Veröffentlichung | Mitglied(er) der Patentfamilie | Datum der Veröffentlichung |
|---|---|---|---|
| DE 102020125743 A1 | 07-04-2022 | CA 3197468 A1 | 07-04-2022 |
| | | DE 102020125743 A1 | 07-04-2022 |
| | | EP 4221569 A1 | 09-08-2023 |
| | | WO 2022069452 A1 | 07-04-2022 |
| DE 102019129288 A1 | 06-05-2021 | CA 3159343 A1 | 06-05-2021 |
| | | DE 102019129288 A1 | 06-05-2021 |
| | | EP 4051089 A1 | 07-09-2022 |
| | | JP 2023500092 A | 04-01-2023 |
| | | US 2022362553 A1 | 17-11-2022 |
| | | WO 2021084005 A1 | 06-05-2021 |

EPO FORM P0461

Für nähere Einzelheiten zu diesem Anhang : siehe Amtsblatt des Europäischen Patentamts, Nr.12/82